Europäisches Patentamt

(19) European Patent Office

Office européen des brevets

(11) Publication number: **0 207 541**
**B1**

# (12) EUROPEAN PATENT SPECIFICATION

(45) Date of publication of the patent specification:
**11.10.89**

(21) Application number: **86200951.1**

(22) Date of filing: **30.05.86**

(51) Int. Cl.⁴: **C07D 301/10, B01J 23/50,**
**B01J 27/10, B01J 23/66,**
**B01J 37/04, B01J 37/24**

(54) Process for the preparation of a silver-containing catalyst.

(30) Priority: **28.06.85  NL 8501865**

(43) Date of publication of application:
**07.01.87 Bulletin 87/2**

(45) Publication of the grant of the patent:
**11.10.89 Bulletin 89/41**

(84) Designated Contracting States:
**BE CH DE FR GB IT LI NL SE**

(56) References cited:
**EP-A- 0 059 422**
**DE-A- 2 925 625**
**FR-A- 2 257 578**
**FR-A- 2 534 581**
**US-A- 2 765 283**
**US-A- 3 894 963**

The file contains technical information submitted after
the application was filed and not included in this
specification

(73) Proprietor: **SHELL INTERNATIONALE RESEARCH**
**MAATSCHAPPIJ B.V., Carel van Bylandtlaan 30,**
**NL-2596 HR Den Haag(NL)**

(72) Inventor: **Boxhoorn, Gosse, Badhuisweg 3, NL-1031 CM**
**Amsterdam(NL)**
Inventor: **Klazinga, Aan Hendrik, Badhuisweg 3,**
**NL-1031 CM Amsterdam(NL)**
Inventor: **Velthuis, Otto Mente, Badhuisweg 3,**
**NL-1031 CM Amsterdam(NL)**

(74) Representative: **Aalbers, Onno et al, P.O. Box 302,**
**NL-2501 CH The Hague(NL)**

ACTORUM AG

## Description

The invention relates to a process for the preparation of a silver-containing catalyst suitable for the preparation of ethylene oxide, to the prepared catalyst and to the use of the catalyst for the preparation of ethylene oxide, and the ethylene oxide thus obtained.

It is generally known for a silver-containing catalyst to be employed in the preparation of ethylene oxide from ethylene. See for example US patent specification No. 2 765 283, British patent specification No. 1413251 and the literature cited therein. In order to obtain improved silver catalysts, efforts have been directed for many years towards modifying the silver catalysts with the aid of promoters. For example, in the above-mentioned British patent specification No. 1413251 is described a process in which a silver compound is applied to a carrier, after which the applied silver compound is reduced to silver and in which additionally a promoter in the form of potassium oxide, rubidium oxide or cesium oxide or a mixture thereof is deposited on the carrier coincidentally with the silver.

The applicant has found new silver catalysts with a higher activity.

The invention relates to a process for the preparation of a silver-containing catalyst suitable for the oxidation of ethylene to ethylene oxide, in which a silver compound is applied to a carrier, after which the silver compound is reduced to metallic silver, which process is characterized in that the carrier has been prepared by mixing an aluminium compound with a chlorine compound and calcining the resulting mixture.

It is observed that the use of hydrogen chloride as peptizing agent in the preparation of aluminium oxide compositions suitable for extrusion is advised against (see US patent specification No. 4,379,134, column 5, line 6). In US patent specifications 3,850,849 and 3,894,963 the use of hydrogen chloride as binding agent in aluminium oxide is, however, recommended.

It is surprising to find that carriers treated with chloride make the silver catalysts more active.

The aluminium compounds can preferably be modifications of aluminium oxide, which when calcined at between 1200 °C and 1700 °C can produce alpha-aluminium oxide, such as gamma-aluminium oxide. Another possibility is to use a hydrated aluminium oxide, such as boehmite, which on calcining at up to 1100 °C can produce gamma-aluminium oxide and on further calcining at temperatures of between 1200 °C and 1700 °C can produce alpha-aluminium oxide.

The chlorine compounds employed in the process according to the invention are in particular hydrogen chloride or trichloroacetic acid, but metal chlorides are also suitable, such as osmium chloride, iridium chloride, germanium chloride, cesium chloride, tin chloride or aluminium chloride.

Preferably, 0.1 to 10 wt% chlorine compound calculated on the weight of the aluminium compound (as $Al_2O_3$) can be mixed with the aluminium compound.

For the preparation of the modified carrier, preferably an aluminium compound is mixed with water and the chlorine compound, the mixture thus obtained being extruded to shaped particles which are subsequently calcined. Calcination can take place in one or more steps, depending on the choice of starting material. In general, sufficient water is added to make the mixture extrudable. The extrudable paste obtained is then extruded in an extruder to form shaped pieces. These shaped pieces are heated, during which water still present is evaporated. The solid pieces are calcined at a temperature of between 1200 °C and 1700 °C. Suitable starting materials are powders of gamma-aluminium oxide, alpha-aluminium oxide monohydrate, alpha-aluminium oxide trihydrate and beta-aluminium oxide monohydrate, which are sintered during the calcination, with fusion of the powder particles taking place.

The effective catalyst surface area can vary from between 0.2 and 5 $m^2/g$.

In order to prepare a catalyst, the modified carrier is impregnated with a solution of a silver compound sufficient to apply, as wished, 1 to 25 weight per cent of silver, calculated on the weight of the total catalyst, to the carrier. The impregnated carrier is separated from the solution and the precipitated silver compound is reduced to silver.

Preferably a promoter is added, for example one or more of the alkali metals: potassium, rubidium or cesium. The promoter can be applied to the carrier before, during or after the impregnation with silver compound. The promoter can also be applied to the carrier after the silver compound has been reduced to silver.

In general, the carrier is mixed with an aqueous solution of a silver salt or silver complex, so that the carrier is impregnated with this solution, after which the carrier is separated from the solution and subsequently dried. The impregnated carrier is then heated to a temperature of between 100 °C and 400 °C for a period necessary for the silver salt (or complex) to decompose and form a finely distributed layer of metallic silver which adheres to the surfaces. A reducing or inert gas can be passed over the carrier during the heating.

Various methods are known for adding the silver. The carrier can be impregnated with an aqueous solution of silver nitrate, then dried, after which the silver nitrate is reduced with hydrogen or hydrazine. The carrier can also be impregnated with an ammoniacal solution of silver oxalate or silver carbonate, the deposition of silver metal being effected by thermally decomposing the salt. Special solutions of a silver salt with certain solubilizing and reducing agents, such as combinations of vicinal alkanolamines, alkyldiamines and ammonia also serve the purpose.

The quantity of added promoter is generally between 20 and 1000 parts by weight of an alkali metal, such as potassium, rubidium or cesium (as metal) per million parts by weight of total catalyst. 50 to 500 parts by weight of alkali metal is particularly suitable. Suitable compounds to serve as starting material for promoters are, for example, nitrates, oxalates, carboxylic acid salts or hydroxides. The most preferred promoter is cesium, the cesium being preferably employed as cesium hydroxide or cesium nitrate.

Some excellent methods are known for adding the alkali metals in which these metals can be applied at the same time as the silver. Suitable alkali metal salts are generally salts which are soluble in the silver-depositing liquid phase. Besides the above-mentioned salts, it is also worth mentioning nitrates, chlorides, iodides, bromides, bicarbonates, acetates, tartrates, lactates and isopropoxides. The use of alkali metal salts which react with the silver present in the solution and thus cause silver salts to be prematurely precipitated from an impregnating solution should, however, be avoided. For example, potassium chloride should not be used for impregnating techniques in which an aqueous silver nitrate solution is used, but potassium nitrate can be used instead. Potassium chloride can be suitably used in a process in which an aqueous solution of silver amine complexes, from which no silver chloride will precipitate, is used.

In addition, the amount of alkali metal deposited on the carrier can be adjusted within certain limits by washing out a part of the alkali metal with, preferably, anhydrous methanol or ethanol. This method is employed subsequently if the concentration of the applied alkali metal is found to be too high. The temperatures, contact times and the drying with gases can be adjusted. Care should be taken to ensure that no traces of alcohol remain in the carrier.

A preferably employed process consists of the carrier being impregnated with an aqueous solution containing both alkali metal salt and silver salt, the impregnating solution being composed of a silver salt of a carboxylic acid, an organic amine, a salt of potassium, rubidium or cesium and an aqueous solvent. For example, a potassium-containing silver oxalate solution can be prepared in two ways. Silver oxide can be reacted with a mixture of ethylene diamine and oxalic acid, giving a solution containing a silver oxalate ethylene diamine complex, to which a certain amount of potassium and possibly other amines such as ethanolamine is added. Silver oxalate can also be precipitated from a solution of potassium oxalate and silver nitrate, the silver oxalate thus obtained then being repeatedly washed in order to remove the attached potassium salts until the desired potassium content is obtained. The potassium-containing silver oxalate is then solubilized with ammonia and/or amine. Solutions containing rubidium and cesium can also be prepared in this way. The thus impregnated carriers are then heated to a temperature of between 100 °C and 400 °C, preferably between 125 °C and 325 °C.

It should be noted that, irrespective of the nature of the silver in the solution before the precipitation onto the carrier, reference is always made to reduction to metallic silver, whereas it could also be referred to as decomposition on heating. It is preferred to think in terms of reduction, since positively charged Ag ions are converted into metallic Ag. The reduction times can be simply adapted to the starting materials employed.

As mentioned above, a promoter is preferably added to the silver. Cesium is the most preferred promoter in view of the fact that its selectivity for ethylene oxide has been found to be the highest in comparison with the use of potassium or rubidium as promoter.

The silver catalysts prepared by the process according to the present invention appear to be particularly active catalysts for the direct catalytic oxidation of ethylene to ethylene oxide with the aid of molecular oxygen. The conditions for carrying out the oxidation reaction in the presence of the silver catalysts according to the invention are fairly similar to those already described in the literature. This applies to, for example, suitable temperatures, pressures, residence times, diluents such as nitrogen, carbon dioxide, steam, argon, methane or other saturated hydrocarbons, the presence or absence of moderating agents to control the catalytic action, for example 1,2-dichloroethane, vinyl chloride or chlorinated polyphenyl compounds, the desirability of employing either recirculating treatments or successive conversions in different reactors to enhance the yield of ethylene oxide, as well as any other special conditions which may be chosen for processes for the preparation of ethylene oxide. Usually, the pressures employed vary from about atmospheric pressure to about 35 bar. Higher pressures are, however, by no means excluded. The molecular oxygen employed as reactant can be obtained from conventional sources. The oxygen supply can consist of substantially pure oxygen, of a concentrated oxygen stream consisting of a large amount of oxygen with smaller amounts of one or more diluents, such as nitrogen argon, etc., or of another oxygen-containing stream, such as air.

In a preferably employed application of the silver catalysts according to the present invention, ethylene oxide is prepared by contacting an oxygen-containing gas that has been separated from air and that contains not less than 95% oxygen with ethylene in the presence of the catalysts in question at a temperature within the range of 210 °C to 285 °C and preferably between 225 °C and 270 °C. Gas hourly space velocities may range from 2800 to 8000 h$^{-1}$.

In the reaction of ethylene with oxygen ethylene oxide, the ethylene is present in at least a double molecular quantity, but the quantity of ethylene employed is generally much higher. The conversion is therefore calculated according to the quantity of converted oxygen in the reaction and we therefore speak of oxygen conversion. This oxygen conversion is dependent on the temperature of the reaction and is a measure of the activity of the catalyst. The values $T_{30}$, $T_{40}$ and $T_{50}$ refer to the temperatures at 30 mol%, 40 mol% and 50 mol% conversion respectively of the oxygen in the reactor. The temperatures are generally higher for a higher conversion and are highly dependent on the catalyst employed and the reaction conditions.

Example 1

21 g of 35 wt% hydrochloric acid was diluted with 77 ml water and mixed with 55 g Kaiser aluminium oxide (26102) $(Al_2O_3.H_2O)$ in 100 g water by adding the solution to the aluminium oxide, and the mixture was kneaded for 15 minutes in a masticator. 220 g $Al_2O_3.H_2O$ and 50 ml water was then added to the kneaded mixture which was kneaded for a further 10 minutes. The resulting paste was extruded and the shaped pieces were dried at 120 °C and subsequently calcined at progressively higher temperatures. Calcination was started with the temperature rising at a rate of 100 °C/h to 500 °C. Calcination was then continued for 2 hours at 500 °C, after which the temperature was raised in 2 hours to 1400 °C. Finally, calcination was continued for 2 hours at 1400 °C. The pore volume of the shaped pieces was 0.58 ml.g⁻¹ and the average pore diameter was 1.4 μm. The resulting shaped pieces were impregnated with an aqueous solution of silver oxalate to which cesium hydroxide was added. The impregnation was carried out for 10 minutes under vacuum, after which the shaped pieces were separated from the solution and placed in a hot air stream at a temperature of 250-270 °C for 10 minutes in order to convert the silver salt to silver. The aqueous solution of silver oxalate was a 28 wt% Ag-containing aqueous solution in which the silver oxalate was complexed with ethylene diamine and to which solution cesium hydroxide was added. After the hot air treatment the thus impregnated shaped pieces contained 21 wt% Ag (calculated on total catalyst) and 290 parts by weight of cesium per million parts by weight of total catalyst.

The catalyst obtained was then used for the preparation of ethylene oxide from ethylene and oxygen. A cylindrical steel reactor with a length of 40 cm and a cross-section of 5 mm was filled entirely with catalyst particles of about 1 mm in size. The reactor was placed in a bath in which silicon oxide/aluminium oxide particles were present in a fluidized bed. A gas mixture with the following composition was passed through the reactor: 30 mol% ethylene, 8.5 mol% oxygen, 7 mol% carbon dioxide, 54,5 mol% nitrogen and 5.5 parts per million parts of gas, of vinyl chloride as moderator. The space velocity was 3300 1.1⁻¹.h⁻¹. The pressure was 15 bar and the temperature was dependent on the set oxygen conversion. The measuring equipment was connect to the reactor and to a computer such that the conversion and the temperature could be accurately controlled. The concentrations of the reaction components were determined with the aid of gas chromatography and mass spectrometry. After 24 h the reaction temperature was measured. The reaction temperature $T_{40}$ was 225 °C for this catalyst. Under the same reaction conditions $T_{40}$ for the standard S839 catalyst was 236 °C.

Examples 2-8

Carriers and catalysts were made in the same way as in Example 1, the catalysts then being tested in the same way in respect of their activity.

These catalysts were compared with the standard S839 catalyst.

The following table lists the inorganic chlorides added to the aluminium oxide and gives the metal/aluminium atom ratio. The table also gives the percentage of silver added and the amount of cesium in ppm (calculated on the weight of the total catalyst) applied to the carrier and the reaction temperature $T_{40}$ in °C.

The same measurements and calculations were carried out for the standard catalyst.

Table

| Example | M/Al atomratio | Ag wt% | Cs (ppm) | $T_{40}$°C |
|---|---|---|---|---|
| 2 AlCl₃ | 0.02 | 14 | 360 | 230 |
| 3 GeCl₄ | 0.001 | 19 | 620 | 222 |
| 4 SnCl₄ | 0.01 | 17 | 390 | 235 |
| 5 SnCl₄ | 0.005 | 15 | 440 | 232 |
| 6 OsCl₃ | 0.01 | 17 | 380 | 229 |
| 7 IrCl₃ | 0.01 | 16 | 390 | 233 |
| 8 CsCl | 0.001 | 14 | 270 | 225 |

Standard S893 catalyst, $T_{40}$ = 236°C.

The tests showed that the catalysts according to the invention were more active than the standard catalyst.

## Claims

1. Process for the preparation of a silver-containing catalyst suitable for the oxidation of ethylene to ethylene oxide, in which a silver compound is applied to a carrier, after which the silver compound is reduced to metallic silver, characterized in that the carrier has been prepared by mixing an aluminium compound with a chlorine compound and by calcining the obtained mixture.

2. Process according to claim 1, characterized in that the chlorine compound is hydrogen chloride.

3. Process according to claim 1, characterized in that the chlorine compound is osmium chloride, iridium chloride, germanium chloride, cesium chloride, tin chloride or aluminium chloride.

4. Process according to one or more of claims 1-3, characterized in that the aluminium compound is mixed with 0.1 to 10 wt% of chlorine compound, calculated on the weight of the aluminium compound.

5. Process according to one or more of claims 1-4, characterized in that the aluminium compound is an aluminium oxide or a hydrate of an aluminium oxide.

6. Process according to claim 5, characterized in that boehmite or gamma-aluminium oxide is employed.

7. Process according to one ore more of claims 1-6, characterized in that the aluminium compound is mixed with water and the chlorine compound, the resulting mixture being extruded to shaped carrier particles which are then calcined.

8. Process according to one or more of claims 1-7, characterized in that the carrier is impregnated with a solution of a silver compound sufficient to apply 1 to 25 weight per cent of silver, calculated on the weight of the total catalyst, to the carrier, the impregnated carrier being separated from the solution and the precipitated silver compound being reduced to silver.

9. Process according to one or more of claims 1-8, characterized in that a promoter is applied to the carrier.

10. Process according to claim 8 or 9, characterized in that besides the silver compound applied to the carrier, a sufficient quantity of one or more compounds of potassium, rubidium or cesium is applied, either simultaneously or not, to the carrier to deposit between 20 and 1000 parts by weight of alkali metal (measured as metal) per million parts by weight of total catalyst.

11. Catalyst prepared with the aid of the process according to claims 1-10.

12. Process for the preparation of ethylene oxide by oxidation of ethylene in the presence of a silver-containing catalyst, characterized in that the process is carried out in the presence of a silver-containing catalyst prepared with the aid of the process according to one or more of claims 1-10 or of a catalyst according to claim 11.

## Patentansprüche

1. Verfahren zur Herstellung eines silberhaltigen Katalysators für die Oxidation von Ethylen zu Ethylenoxid, bei dem eine Silberverbindung auf einem Träger aufgebracht und anschließend die Silberverbindung zu metallischem Silber reduziert wird, dadurch gekennzeichnet, daß der Träger hergestellt worden ist durch Mischen einer Aluminiumverbindung mit einer Chlorverbindung und Brennen der erhaltenen Mischung.

2. Verfahren nach Anspruch 1, dadurch gekennzeichnet, daß die Chlorverbindung Chlorwasserstoff ist.

3. Verfahren nach Anspruch 1, dadurch gekennzeichnet, daß die Chlorverbindung Osmiumchlorid, Iridiumchlorid, Germaniumchlorid, Caesiumchlorid, Zinnchlorid oder Aluminiumchlorid ist.

4. Verfahren nach einem der Ansprüche 1 bis 3, dadurch gekennzeichnet, daß die Aluminiumverbindung mit 0,1 bis 10 Gew.-% Chlorverbindung, bezogen auf das Gewicht der Aluminiumverbindung, vermischt wird.

5. Verfahren nach einem oder mehreren der Ansprüche 1 bis 4, dadurch gekennzeichnet, daß die Aluminiumverbindung Aluminiumoxid oder -oxidhydrat ist.

6. Verfahren nach Anspruch 5, dadurch gekennzeichnet, daß Boehmit oder γ-Aluminiumoxid verwendet wird.

7. Verfahren nach einem oder mehreren der Ansprüche 1 bis 6, dadurch gekennzeichnet, daß die Aluminiumverbindung mit Wasser und der Chlorverbindung gemischt wird, das erhaltene Gemisch zu Trägerteilchen geformt wird und diese dann gebrannt werden.

8. Verfahren nach einem oder mehreren der Ansprüche 1 bis 7, dadurch gekennzeichnet, daß der Träger mit einer Lösung einer Silberverbindung für die Aufbringung von 1 bis 25 Gew.-% Silber, bezogen auf das Gesamtgewicht des Katalysators, imprägniert wird, der imprägnierte Träger aus der Lösung genommen und die ausgefällte Silberverbindung zu Silber reduziert wird.

9. Verfahren nach einem oder mehreren der Ansprüche 1 bis 8, dadurch gekennzeichnet, daß auf den Träger ein Promotor aufgebracht wird.

10. Verfahren nach Anspruch 8 oder 9, dadurch gekennzeichnet, daß außer der Silberverbindung eine ausreichende Menge von einer oder mehreren Verbindung(en) von Kalium, Rubidium oder Caesium aufgebracht wird, und zwar gegebenenfalls gleichzeitig, um 20 bis 1000 ppm Alkalimetall, bezogen auf das Gesamtgewicht des Katalysators, abzuscheiden.

11. Katalysator, hergestellt nach dem Verfahren der Ansprüche 1 bis 10.

12. Verfahren zur Herstellung von Ethylenoxid durch Oxidation von Ethylen in Gegenwart eines silberhaltigen Katalysators, dadurch gekennzeichnet, daß das Verfahren in Gegenwart eines silberhaltigen Katalysators durchgeführt wird, der hergestellt worden ist nach einem oder mehreren der Ansprüche 1 bis 10 oder mit Hilfe eines Katalysators nach Anspruch 11.

**Revendications**

1. Procédé pour la préparation d'un catalyseur contenant de l'argent qui convient pour l'oxydation de l'éthylène en oxyde d'éthylène, dans lequel on applique un composé de l'argent sur un support et on réduit ensuite le composé de l'argent en argent métallique, caractérisé en ce que l'on a préparé le support en mélangeant un composé aluminique avec un composé du chlore et en calcinant le mélange obtenu.

2. Procédé selon la revendication 1, caractérisé en ce que le composé du chlore est le chlorure d'hydrogène.

3. Procédé selon la revendication 1, caractérisé en ce que le composé du chlore est le chlorure d'osmium, le chlorure d'iridium, le chlorure de germanium, le chlorure de césium, le chlorure d'étain ou le chlorure d'aluminium.

4. Procédé selon l'une quelconque ou plusieurs des revendications 1 à 3, caractérisé en ce que l'on mélange le composé aluminique avec 0,1 à 10% en poids de composé du chlore, calculé sur le poids du composé aluminique.

5. Procédé selon l'une quelconque ou plusieurs des revendications 1 à 4, caractérisé en ce que le composé aluminique est un oxyde d'aluminium ou un hydrate d'un oxyde d'aluminium.

6. Procédé selon la revendication 5, caractérisé en ce que l'on utilise la boehmite ou le gamma-oxyde d'aluminium.

7. Procédé selon l'une quelconque ou plusieurs des revendications 1 à 6, caractérisé en ce que l'on mélange le composé aluminique avec de l'eau et le composé du chlore, le mélange résultant étant extrudé en particules façonnées de support qui sont ensuite calcinées.

8. Procédé selon l'une quelconque ou plusieurs des revendications 1 à 7, caractérisé en ce que l'on imprègne le support avec une solution d'un composé de l'argent suffisante pour appliquer sur le support 1 à 25% en poids d'argent, calculé sur le poids du catalyseur total, le support imprégné étant séparé de la solution et le composé de l'argent précipité étant réduit en argent.

9. Procédé selon l'une quelconque ou plusieurs des revendications 1 à 8, caractérisé en ce que l'on applique un promoteur sur le support.

10. Procédé selon la revendication 8 ou 9, caractérisé en ce qu'en plus du composé de l'argent appliqué sur le support, on applique une quantité suffisante d'un ou plusieurs composés du potassium, du rubidium ou du césium, que ce soit simultanément ou non, sur le support afin de déposer entre 20 et 1000 parties en poids du métal alcalin (mesurées par rapport au métal) par million de parties en poids du catalyseur total.

11. Catalyseur préparé à l'aide du procédé selon les revendications 1 à 10.

12. Procédé pour la préparation d'oxyde d'éthylène par oxydation de l'éthylène en présence d'un catalyseur contenant de l'argent, caractérisé en ce que l'on effectue le procédé en présence d'un catalyseur contenant de l'argent préparé grâce au procédé selon l'une quelconque ou plusieurs des revendications 1 à 10 ou d'un catalyseur selon la revendication 11.